(19) Europäisches Patentamt
European Patent Office
Office européen des brevets

(11) Veröffentlichungsnummer : **0 398 165 B1**

(12) # EUROPÄISCHE PATENTSCHRIFT

(45) Veröffentlichungstag der Patentschrift :
**04.08.93 Patentblatt 93/31**

(51) Int. Cl.[5] : **A61K 31/495,** // (A61K31/495, 31:445, 31:175, 31:13)

(21) Anmeldenummer : **90108858.3**

(22) Anmeldetag : **11.05.90**

(54) **Kombinationspräparate enthaltend Rifampicin und Thioacetazon sowie gegebenenfalls Isonicotinsäurehydrazid oder Ethambutol als aktive Wirkstoffe.**

(30) Priorität : **19.05.89 DE 3916417**

(43) Veröffentlichungstag der Anmeldung :
**22.11.90 Patentblatt 90/47**

(45) Bekanntmachung des Hinweises auf die Patenterteilung :
**04.08.93 Patentblatt 93/31**

(84) Benannte Vertragsstaaten :
**AT BE CH DE DK ES FR GB GR IT LI LU NL SE**

(56) Entgegenhaltungen :
**AM. REV. RESP. DIS., Band 125, 1982; F.M. COLLINS et al., Seiten 58-60.**
**THE INTERNATIONAL JOURNAL OF TUBERCULOSIS, Band 70, Nr. 1, 1989, Longman Group UK Ltd, GB; A.AZIZ et al., Seiten 45-51.**

(56) Entgegenhaltungen :
**CHEMICAL ABSTRACTS, Band 79, Nr. 13, 01 Oktober 1973, Columbus, OH (US); O.O.MAKEEVA, Seite 81, Nr. 74133y.**
**AM. REV. RESP. DIS., Band 133, 1986; D.A.MITCHISONet al., Seiten 423-430.**
**J. AM. ACAD. DERMATOL., Band 19, 1988; S.K.HIRA et al., Seiten 451-457.**

(73) Patentinhaber : **FATOL ARZNEIMITTEL GMBH**
**Robert-Koch-Strasse, Postfach 1260**
**W-6685-Schiffweiler (DE)**

(72) Erfinder : **Schaper, Klaus Jürgen, Dr.**
**Parkallee 39c**
**W-2061 Borstel (DE)**
Erfinder : **Seydel, Joachim, Prof.**
**Mühloh 2**
**W-2061 Borstel (DE)**

(74) Vertreter : **UEXKÜLL & STOLBERG**
**Patentanwälte**
**Beselerstrasse 4**
**W-2000 Hamburg 52 (DE)**

**EP 0 398 165 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Kombinationspräparate, die Rifampicin und Thioacetazon sowie gegebenenfalls Isonicotinsäurehydrazid oder Ethambutol als aktive Wirkstoffe enthalten. Die erfindungsgemäßen Kombinationspräparate haben sich bei der Behandlung mykobakterieller Erkrankungen als außerordentlich wirksam erwiesen. Die Präparate entfalten insbesondere therapeutische Wirksamkeit gegenüber Mykobakterien des MOTT-Komplexes (mykobacteria other than tuberculosis) und unter diesen wiederum gegenüber der Gruppe der aviären Mykobakterien.

Das Spektrum der mykobakteriellen Erkrankungen reicht von der "klassischen" durch Mykobakterium tuberculosis hervorgerufenen Tuberkulose über die Lepra bis zu den durch sogenannte atypische oder "nicht-tuberkulose" Mykobakterien verursachten Infektionen. Letztere werden häufig auch unter der Bezeichnung "MOTT-Komplex" zusammengefaßt. Diese Arten sind zwar weniger virulent und pathogen als M.tuberculosis oder M.leprae, sie gewinnen jedoch bei Patienten mit geschwächtem Immunsystem zunehmend an Bedeutung. Diese Situation liegt vor allem bei Patienten mit AIDS und Alterstuberkulosen, jedoch auch nach Organtransplantationen vor, und es wird ganz generell ein Anstieg der Infektionen mit atypischen Mykobakterien beobachtet.

Bei AIDS-Patienten sind Infekte mit Mykobakterien des MOTT-Komplexes und insbesondere mit M.avium häufig für den lethalen Ausgang der Krankheit zumindest mitverantwortlich (vgl.Ärztliche Praxis 13, 370 (1989)).

In diesem Zusammenhang hat es sich als verhängnisvoll erwiesen, daß bisher keine wirklich wirksame Therapie der durch atypische Mykobakterien hervorgerufenen opportunistischen Infekte zur Verfügung steht.

Es ist daher Aufgabe der Erfindung, Präparate zu schaffen, mit deren Hilfe durch Mykobakterien hervorgerufene Infektionen, und insbesondere die Mykobakterien des MOTT-Komplexes wie M.avium wirkungsvoll bekämpft werden können.

Zur Lösung der Aufgabe werden die Kombinationspräparate nach den Ansprüchen 1 bis 9 vorgeschlagen.

Erfindungsgemäß hat es sich überraschenderweise gezeigt, daß mit Kombinationspräparaten, die Rifampicin (RMP) und Thioacetazon (Thz) in Kombination enthalten, sowohl das Wachstum und die Vermehrung der typischen Mykobakterien M.tuberculosis und M.leprae als auch dasjenige der atypischen Mykobakterien des MOTT-Komplexes und insbesondere von M.avium außerordentlich stark gehemmt werden können. Es tritt dementsprechend ein therapeutischer Effekt ein, wie er durch die Einzelkomponenten des erfindungsgemäßen Kombinationspräparates mit therapeutisch vertretbaren Dosismengen nicht erreicht werden kann.

Zwar gilt RMP derzeit als das wirksamste Mittel gegen Mykobakterien, allein entfaltet es jedoch gegenüber den Mykobakterien des MOTT-Komplexes keine oder nur geringe Wirksamkeit (vgl. Beispiel 2, Tabelle 2 sowie Tabelle 5). Ferner treten immer wieder Stämme von M.tuberculosis und M.leprae in Erscheinung, die entweder primär resistent gegen RMP sind, oder aber unter der Monotherapie durch Selektion Resistenz erworben haben.

Die antimykobakterielle Wirkung von Thz ist ebenfalls seit langem bekannt. Es ist jedoch allein in verträglichen Dosen nur schwach wirksam und kann bei höheren Dosen zu unerwünschten Nebenwirkungen führen.

Unerwarteterweise hat sich nunmehr gezeigt, daß die Kombination von RMP und Thz weit überdurchschnittliche synergistische Wirksamkeit gegenüber Mykobakterien und insbesondere gegenüber M.avium aufweist. Während beispielsweise die mittlere Hemmkonzentration von Thz allein gegenüber einem aus einem AIDS-Patienten isolierten M.avium-Stamm (M.avium 227, vgl. Beispiel 2) 32 µg/ml und diejenige von Rifampicin 8µg/ml beträgt, sind jeweils nur 0,36 µg/ml der beiden Komponenten in Kombination erforderlich, um die Vermehrung der Bakterienzellen vollständig zu hemmen (vgl. Tabelle 3). Es handelt sich demgemäß nicht um einen additiven, sondern um einen echten und sehr stark ausgeprägten synergistischen Effekt. Dieses Ergebnis ist umso überraschender, als in der antimykobakteriellen Therapie vom "fulldose-Prinzip" ausgegangen wird, d.h. eine Wirksamkeit der Einzelkomponenten wird in der Kombination nur dann erwartet, wenn die volle einzeln wirksame Dosierungsmenge zur Anwendung kommt.

Gemäß einer besonders bevorzugten Ausführungsform der Erfindung enthalten die Kombinationspräparate zusätzlich Isonicotinsäurehydrazid (INH) oder Ethambutol (EMB). Erfindungsgemäß hat es sich nämlich überraschenderweise gezeigt, daß in Gegenwart von INH oder EMB die für eine erfolgreiche Hemmung der Zielorganismen erforderlichen Dosen von RMP und Thz nochmals drastisch sinken (vgl. Beispiel 5, Tabelle 5). Die Gesamt-Wirkstoffmenge kann demgemäß durch das Hinzutreten von INH oder EMB nochmals deutlich vermindert werden.

Die erfindungsgemäßen Kombinationspräparate müssen aufgrund der Natur der in Betracht kommenden Erkrankungen im allgemeinen über längere Zeiträume, d.h. häufig etwa 12 bis 24 Monate lang, verabreicht werden. Da der therapeutische Effekt nur dann eintritt, wenn beide Substanzen gleichzeitig appliziert werden, muß gewährleistet sein, daß die gemeinsame Einnahme regelmäßig und in den vorgeschriebenen Mengen erfolgt. Bei einer Langzeittherapie mit den einzelnen Substanzen ist dies jedoch nicht gewährleistet.

Die erfindungsgemäßen Kombinationspräparate liegen demgemäß vorzugsweise als fixe Kombination vor, wobei in der Kombination das Verhältnis der Wirkstoffe RMP zu Thz vorzugsweise (2 bis 6) : (0,5 bis 2) beträgt. In Gegenwart von INH oder EMB als zusätzlichem Wirkstoff in dem erfindungsgemäßen Kombinationspräparat beträgt das Verhältnis von RMP zu Thz zu INH vorzugsweise (2 bis 6) : (0,5 bis 2) : (1 bis 4) und das Verhältnis von RMP zu Thz zu EMB (2 bis 6) : (0,5 bis 2) : (6 bis 16).

In besonders bevorzugter Weise liegt das Präparat in einer zur oralen Verabreichung geeigneten Form, d.h. als Tabletten, Dragees oder Kapseln vor. Derartige Dosierungseinheiten enthalten vorzugsweise 300 bis 700 mg RMP und 50 bis 100 mg Thz, und in besonders bevorzugter Weise 400 mg RMP und 75 mg Thz. Ist zusätzlich INH oder EMB vorhanden, so sind Dosierungseinheiten bevorzugt, die 100 bis 300 mg RMP, 25 bis 75 mg Thz und 50 bis 200 mg INH oder 400 bis 700 mg EMB enthalten.

Die Herstellung der Tabletten kann in üblicher Weise unter Einbeziehung von herkömmlichen Tablettierungs-, Träger- und Hilfsstoffen erfolgen.

Die Erfindung wird nachfolgend anhand von Beispielen erläutert.

Beispiel 1

Isolierung von M.avium aus Patientenseren

Aus den Seren von vier verschiedenen Patienten wurden nach bekannten Verfahren ("Isolierung von Mykobakterien", DIN 58943, Teil 3, 1980)) jeweils Stämme von Mykobakterium avium isoliert.

Das Krankheitsbild des jeweiligen Patienten und die Bezeichnung des Isolats sind nachfolgend in Tabelle 1 wiedergegeben.

Tabelle 1

| Erkrankung | Bezeichnung |
|---|---|
| Atypische Mykobakteriose bei AIDS | M.avium 227 |
| Atypische Mykobakteriose | M.avium Ro |
| " | M.avium Dan |
| " | M.avium Bi |

Beispiel 2

Bestimmung der Minimalen Hemmkonzentration (MHK) der Einzelsubstanzen.

Die MHK der Einzelsubstanzen RMP und Thz wurde nach bekannten Standardverfahren ("Methoden zur Empfindlichkeitsprüfung von bakteriellen Krankheitserregern", DIN 58940, Teil 5) gegenüber den M.avium-Stämmen gemäß Beispiel 1 bestimmt.

Die Bakterien wurden in flüssigem Nährboden nach Lockemann (vgl. "Nachweisverfahren für Mykobakterien aus Untersuchungsmaterial, III, Nährbodenrezepte zur Kultur von Tuberkulosebakterien, 1978, Herausgeber: Deutsches Zentralkomitee zur Bekämpfung der Tuberkulose, Poppenhusenstraße 14c, 2000 Hamburg 60) angereichert mit 0,5% Albumin und 1% Humanserum angezogen. Jeweils 5 x $10^{-3}$ bis 5 x $10^{-5}$ mg bezogen auf Feuchtgewicht, der Mykobakterienstämme wurden in 2 ml der Kulturflüssigkeit bei 37°C mit abgestuften Konzentrationen von RMP bzw. Thz inkubiert. Die Ablesung der Kulturröhrchen erfolgte nach 8 bis 15 Tagen.

Die MHK ist diejenige Konzentration des Hemmstoffes in µg/ml, bei der keine Vermehrung der eingesäten Zellen festzustellen ist.

Die Ergebnisse sind in Tabelle 2 wiedergegeben. Sie zeigen, daß M.avium Ro vollständig resistent gegenüber beiden Substanzen ist, während M.avium Dan resistent gegenüber Thz ist und 16 µg/ml RMP allein erforderlich sind, um das Wachstum vollständig zu hemmen. Für die übrigen untersuchen Stämme liegt die entsprechende Menge für RMP zwischen 3 und 8 µg/l und für Thz zwischen 3 und 32 µg/l. Auch diese Stämme sind jedoch durch die Einzelpräparate nicht therapierbar, da die erforderlichen Serumkonzentrationen bei klinisch üblicher und vertretbarer Dosierung nicht erreicht werden.

## Tabelle 2

| Stamm | MHK µg/ml | |
|---|---|---|
| | RMP | Thz |
| M.avium 227 | 8 | 32 |
| M.avium Ro | > 32 | > 32 |
| M.avium Dan | 16 | > 32 |
| M.avium Bi | 3 | 3 |

### Beispiel 3

Die MHK-Werte der erfindungsgemäßen Kombinationen gegenüber den Stämmen gemäß Beispiel 1 wurden durch Verdünnungsreihen mit konstanten Wirkstoffverhältnissen bestimmt.

Die Bakterien wurden unter den Bedingungen gemäß Beispiel 2 inkubiert.

Die Summe der fraktionellen Hemmkonzentration (FII) wurde nach der Gleichung von C.W. Norden et al., J. Infect.-Dis. 140, 290 (1978) berechnet:

$$\text{FII} = \frac{\text{MHK der Verbindung A in Kombination}}{\text{MHK der Verbindung A allein}} + \frac{\text{MHK der Verbindung B in Kombination}}{\text{MHK der Verbindung A allein}}$$

Bei $FII<1$ liegt Synergismus vor, während $FII>>1$ Antagonismus bedeutet.

Die Ergebnisse sind in Tabelle 3 wiedergegeben. Sie zeigen, daß in allen Fällen FII-Werte erhalten wurden, die weit unter 1 liegen. Die Wirkstoffe RMP und Thz weisen demgemäß in Kombination eine extreme synergistische Wirksamkeit gegenüber den untersuchten Stämmen auf.

## Tabelle 3

| Stamm | | MHK µg/ml (komb.) RMP/Thz | FII |
|---|---|---|---|
| M.avium 227 | | 0,36/0,36 | 0,056 |
| M.avium Ro | | 0,32/0,32 | < 0,01 |
| M.avium Dan | | 0,24/0,24 | < 0,022 |
| M.avium Bi | a. | 0,22/0,22 | 0,146 |
| | b. | 0,18/0,18 | 0,120 |

### Beispiel 4

Die MHK-Werte der erfindungsgemäßen Kombination aus RMP und Thz wurden zusätzlich durch Schachbrett-Titrationen (vgl. Berenbaum, N.C., J. Infekt. Dis. 137, 122 bis 130, 1978 und J.K. Seydel et al., Chemotherapie 29, 249 (1983)) gegenüber dem Stamm M. tuberculosis H37Rv sowie gegenüber dem RMP-resistenten Patientenstamm M. avium BK ermittelt.

Die Ergebnisse sind in Tabellen 4a und 4b wiedergegeben. Sie zeigen, daß die synergistische Wirksamkeit der Kombination auch gegenüber diesen Stämmen zum Ausdruck kommt.

Beispiel 5

Die Versuche gemäß den Beispielen 2 und 3 wurden wiederholt, indem die MHK-Werte der Wirkstoffe RMP, Thz, INH und EMB allein sowie der erfindungsgemäßen Kombinationen RMP/Thz, RMP/Thz/INH und RMP/Thz/EMP gegenüber 10 weiteren, aus verschiedenen Patienten isolierten Mykobakterien des Avium-Komplexes bestimmt wurden.

Die Ergebnisse sind in Tabelle 5 wiedergegeben. Sie zeigen die synergistische Wirksamkeit der erfindungsgemäßen Kombinationen, wobei in allen Fällen durch das Hinzutreten von INH oder EMP eine extreme Steigerung des synergistischen Effektes erzielt wurde.

**Beispiel 6**

Aus folgenden Bestandteilen wurden nach herkömmlichen Tablettierverfahren Tabletten hergestellt:

| | a) mg | b) mg | c) mg |
|---|---|---|---|
| Rifampicin | 400 | 300 | 300 |
| Thioacetazon | 75 | 75 | 75 |
| Isonicotinsäurehydrazid | - | 175 | - |
| Ethambutol | - | - | 400 |
| Cellulosepulver | 188 | 163 | 335 |
| Carboxymethyl-Cellulose-Na, quervernetzt | 30 | 30 | 50 |
| $SiO_2$ | 8 | 8 | 10 |
| Talcum | 16 | 16 | 20 |
| Polyvinylpyrrolidon | 8 | 8 | 10 |
| Gesamtmenge | 725 | 775 | 1200 |

## Tabelle 4a

Stamm: H37Rv M. tub.
Medium: Lockemann + 0,5% Albumin + 1% H-Serum
Beimpfung: $1.91 \times 10^{-1}$

Thioacetazon

| Rifampicin ug/ml | .50 | .45 | .40 | .35 | .30 | .25 | .20 | .15 | .10 | .05 | 0 |
|---|---|---|---|---|---|---|---|---|---|---|---|
| .06 | | | | | | | | | | | - |
| .054 | | | | | | | | | | | - |
| .048 | | | | | | | | | | | + |
| .042 | | | | | | | | | | | + |
| .036 | | | | | | | | | | | + |
| .030 | | | | | | | | | | | ++ |
| .024 | | | | | | | | | | | +++ |
| .018 | | | | | | | | | | + | +++ |
| .012 | | | | | | | | | | ++ | +++ |
| .006 | | | | | | | | | + | ++ | +++ |
| 0 | +++ | +++ | +++ | +++ | +++ | +++ | +++ | +++ | +++ | +++ | +++ |

(+++) volle Bakterienvermehrung
(++) abgeschwächte "
(+) stark abgeschwächte "
( ) = (-) keine Vermehrung

## Tabelle 4b

Stamm: M. avium BK.

Medium: Lockemann + 0,5% Albumin + 1% H-Serum

Beimpfung: 1.91 x $10^{-1}$

| | Rifampicin | | | | | | | | | | | |
|---|---|---|---|---|---|---|---|---|---|---|---|---|
| | ug/ml | 8 | 7.2 | 6.4 | 5.4 | 4.8 | 4 | 3.2 | 2.4 | 1.6 | 0.8 | 0 |
| Thioacetazon | 8 | | | | | | | | | | | + |
| | 7.2 | | | | | | | | | | | + |
| | 6.4 | | | | | | | | | | | + |
| | 5.6 | | | | | | | | | | | + |
| | 4.8 | | | | | | | | | | | ++ |
| | 4 | | | | | | | | | | | ++ |
| | 3.2 | | | | | | | | | | | ++ |
| | 2.4 | | | | | | | | | | | ++ |
| | 1.6 | | | | | | | | | | | +++ |
| | 0.8 | | | | | | | | | | | +++ |
| | 0.000 | - | - | - | - | - | - | ++ | ++ | +++ | +++ | +++ |

(+++) volle Bakterienvermehrung

(++) abgeschwächte "

(+) stark abgeschwächte "

( ) = (-) keine Vermehrung

**Tabelle 5**

| Patienten-Code / Verabreichungsform | Substanz | Sa | Me | Kü | Wi | Vo | Bi | Ma | Ka-B | LB | LA |
|---|---|---|---|---|---|---|---|---|---|---|---|
| | | M H K (ug/ml) | | | | | | | | | |
| einzeln | RMP | 8 | 16 | 32 | >32 | 16 | 4 | >32 | >32 | 32 | >32 |
| " | Thz | 32 | 32 | 32 | 32 | 32 | >32 | >32 | >32 | 32 | 16 |
| " | INH | 32 | 16 | 4 | 16 | 8 | >32 | >32 | 32 | 16 | 4 |
| " | EMB | >32 | 8 | >32 | >32 | 4-8 | 4 | 32 | >32 | 16-32 | >32 |
| in Kombination | RMP/ | 4 | 8 | 2 | 4 | 8 | - | 1 | 8 | ≥8 | 2 |
| | Thz | 1 | 2 | 0.5 | 1 | 2 | - | 0.25 | 2 | ≥2 | 0.5 |
| " | RMP/ | 4 | 2 | 0.5 | 2 | 4 | 1 | 2 | 4 | 4 | 2 |
| | Thz/ | 1 | 0.5 | 0.12 | 0.5 | 1 | 0.25 | 1 | 1 | 1 | 1 |
| | INH | 2 | 1 | 0.25 | 1 | 2 | 0.5 | 0.5 | 2 | 2 | 0.5 |
| " | RMP/ | 1 | 1 | 1 | 1 | 4 | - | 1 | 8 | 8 | 2 |
| | Thz/ | 0.5 | 0.5 | 0.5 | 0.5 | 1 | - | 0.5 | 2 | 2 | 0.5 |
| | EMB | 1 | 1 | 1 | 1 | 2 | - | 1 | 4 | 4 | 4 |

## Patentansprüche

1. Kombinationspräparat enthaltend Rifampicin und Thioacetazon als aktive Wirkstoffe.

2. Kombinationspräparat nach Anspruch 1, dadurch gekennzeichnet, daß es die Wirkstoffe im Verhältnis (2 bis 6) : (0,5 bis 2) enthält.

3. Kombinationspräparat nach den Ansprüchen 1 oder 2, dadurch gekennzeichnet, daß es je Dosierungseinheit 300 bis 700 mg Rifampicin und 50 bis 100 mg Thioacetazon enthält.

4. Kombinationspräparat nach den Ansprüchen 1 bis 3, dadurch gekennzeichnet, daß es zusätzlich Isonicotinsäurehydrazid oder Ethambutol enthält.

5. Kombinationspräparat nach Anspruch 4, dadurch gekennzeichnet, daß es die Wirkstoffe Rifampicin, Thioacetazon und Isonicotinsäurehydrazid im Verhältnis (2 bis 6) : (0,5 bis 2) : (1 bis 4) enthält.

6. Kombinationspräparat nach Anspruch 5, dadurch gekennzeichnet, daß es je Dosierungseinheit 100 bis 300 mg Rifampicin, 25 bis 75 mg Thioacetazon und 50 bis 200 mg Isonicotinsäurehydrazid enthält.

7. Kombinationspräparat nach anspruch 4, dadurch gekennzeichnet daß es die Wirkstoffe Rifampicin, Thioacetazon und Ethambutol im Verhältnis (2 bis 6) : (0,5 bis 2) : (6 bis 16) enthält.

8. Kombinationspräparat nach Anspruch 7, dadurch gekennzeichnet, daß es je Dosierungseinheit 100 bis 300 mg Rifampicin, 25 bis 75 mg Thioacetazon und 400 bis 700 mg Ethambutol enthält.

9. Kombinationspräparat nach den Ansprüchen 1 bis 8, dadurch gekennzeichnet, daß es in Tabletten- oder Kapselform vorliegt und die Wirkstoffe gemeinsam mit üblichen Hilfs-und Trägerstoffen enthält.

10. Verwendung der Kombinationspräparate nach den Ansprüchen 1 bis 9 zur Herstellung eines Arzneimittels zur Behandlung mykobakterieller Erkrankungen.

11. Verwendung nach Anspruch 10 zur Herstellung eines Arzneimittels zur Behandlung von Erkrankungen hervorgerufen durch Mykobakterien des MOTT-Komplexes.

12. Verwendung nach Anspruch 11 zur Herstellung eines Arzneimittels zur Behandlung von Erkrankungen hervorgerufen durch Mykobakterien der Gattung avium.

## Claims

1. Combination preparation containing rifampicin and thioacetazone as active ingredients.

2. Combination preparation according to claim 1, characterized in that it contains the active ingredients in the ratio (2 to 6) : (0,5 to 2).

3. Combination preparation according to claims 1 or 2, characterized in that it contains 300 to 700 mg rifampicin and 50 to 100 mg thioacetazone per dosage unit.

4. Combination preparation according to claims 1 to 3, characterized in that it additionally contains isonicotinic acid hydrazide or ethambutol.

5. Combination preparation according to claim 4, characterized in that it contains the active ingredients rifampicin, thioacetazone and isonicotinic acid hydrazide in the ratio (2 to 6) : (0,5 to 2) : (1 to 4).

6. Combination preparation according to claim 5, characterized in that it contains 100 to 300 mg rifampicin, 25 to 75 mg thioacetazone and 50 to 200 mg isonicotinic acid hydrazide per dosage unit.

7. Combination preparation according to claim 4, characterized in that it contains the active ingredients rifampicin, thioacetazone and ethambutol in the ratio (2 to 6) : (0,5 to 2) : (6 to 16).

8. Combination preparation according to claim 7, characterized in that it contains 100 to 300 mg rifampicin, 25 to 75 mg thioacetazone and 400 to 700 mg ethambutol per dosage unit.

**9.** Combination preparation according to claims 1 to 8, characterized in that it is in form of a tablet or capsule and that it contains the active ingredients together with common inactive and carrier substances.

**10.** Use of the combination preparations according to claims 1 to 9 for the preparation of a medicament for the treatment of mykobacterial diseases.

**11.** Use according to claim 10 for the preparation of a medicament for the treatment of diseases caused by mykobacteria of the MOTT-Complex.

**12.** Use according to claim 11 for the preparation of a medicament for the treatment of diseases caused by mykobacteria of the species avium.

## Revendications

**1.** Préparation combinée contenant comme agents actifs de la rifampicine et de la thioacétone.

**2.** Préparation combinée selon la revendication 1, caractérisée en ce qu'elle contient les agents actifs selon la proportion (2 à 6) : (0,5 à 2).

**3.** Préparation combinée selon les revendications 1 et 2, caractérisée en ce qu'elle contient pour chaque l'unité de dosage 300 à 700 mg rifampicine et 50 à 100 mg thioacétone.

**4.** Préparation combinée selon les revendications 1 à 3, caractérisée en ce qu'elle contient de plus de l'isoniacine hydrazide ou de l'éthambutol.

**5.** Préparation combinée selon la revendication 4, caractérisée en ce qu'elle contient les agents actifs rifampicine, thioacétone et isoniacine hydrazide selon la proportion (2 à 6) : (0,5 à 2) : (1 à 4).

**6.** Préparation combinée selon la revendication 5, caractérisée en ce qu'elle contient pour chaque l'unité de dosage 100 à 300 mg rifampicine, 25 à 75 mg thioacétone et 50 à 200 mg isoniacine hydrazide.

**7.** Préparation combinée selon la revendication 4, caractérisée en ce qu'elle contient les agents actifs rifampicine, thioacétone et éthambutol selon la proportion (2 à 6) : (0,5 à 2) : (6 à 16).

**8.** Préparation combinée selon la revendication 7, caractérisée en ce qu'elle contient pour chaque l'unité de dosage 100 à 300 mg rifampicine, 25 à 75 mg thioacétone et 400 à 700 mg ethambutol.

**9.** Préparation combinée selon les revendications 1 à 8, caractérisée en ce qu'elle est présentée sous forme de tablette ou de capsule et contient les agents actifs accompagnés des matériaux de support et auxiliaires habituels.

**10.** Utilisation des préparations combinées selon les revendications 1 à 9 pour la fabrication d'un médicament destiné au traitement de maladies mycobactérielles.

**11.** Utilisation selon la revendication 10 pour la fabrication d'un médicament destiné au traitement de maladies provoquées par des mycobacteries de l'ensemble de MOTT.

**12.** Utilisation selon la revendication 11 pour la fabrication d'un médicament destiné au traitement de maladies provoquées par des mycobactéries du genre avium.